# EUROPEAN PATENT SPECIFICATION

(11) **EP 1 554 936 B1**
(45) Date of publication and mention of the grant of the patent: **23.05.2007**
(21) Application number: 03727526.0
(22) Date of filing: 29.05.2003
(51) Int. Cl.: A23K 1/16, A61K 31/192, A61P 29/00

(54) **KETOPROFEN-SUPPLEMENTED ANIMAL FEED AND USE THEREOF IN THE SIMULTANEOUS TREATMENT OF A GROUP OF ANIMALS FOR PROCESSES WHICH ARE ACCOMPANIED BY FEVER, INFLAMMATION AND/OR PAIN**
TIERFUTTER MIT KETOPROFEN-ZUSATZ UND SEINE VERWENDUNG BEI DER GLEICHZEITIGEN BEHANDLUNG EINER GRUPPE VON TIEREN FÜR VORGÄNGE, DIE MIT FIEBER, ENTZÜNDUNG UND/ODER SCHMERZEN EINHERGEHEN
ALIMENT POUR ANIMAUX ENRICHI EN KETOPROFENE ET SON UTILISATION DANS LE TRAITEMENT D'ETATS PATHOLOGIQUES QUI SE MANIFESTENT PAR DE LA FIEVRE, UNE INFLAMMATION ET/OU UNE DOULEUR DANS UN COLLECTIF ANIMAL, SIMULTANEMENT

(30) Priority: 29.05.2002 ES 200201236
(43) Date of publication of application: 20.07.2005
(73) Proprietor: LABORATORIOS DEL DR. ESTEVE, S.A., 08041 Barcelona (ES)
(72) Inventor: HOMEDES BEGUER, Josep, 08041 BARCELONA (ES); SOLANAS IBARRA, Pedro, Juan, 08041 BARCELONA (ES); LOPEZ CABRERA, Antonio, 08041 BARCELONA (ES); LIZCANO GARCIA, Javier, 08041 BARCELONA (ES)
(74) Representative: Carpintero Lopez, Francisco
(86) International application number: PCT/ES2003/000257
(87) International publication number: WO 2003/101217

(56) References cited:
- EP-A- 0 482 919
- EP-A- 1 234 508
- WO-A-96/18298
- WO-A-97/45113
- ES-T- 2 181 320
- US-A- 3 147 120
- US-A1- 2002 169 212

## Description

### FIELD OF THE INVENTION

The invention relates to the simultaneous oral treatment of Porcine Respiratory Diseases Complex (PRDC) in pigs and Bovine Respiratory Syndrome (BRS) in calves, in a herd of animals, by using a feed supplemented with ketoprofen meant to be simultaneously ingested by a herd of animals.

### BACKGROUND OF THE INVENTION

Ketoprofen [2-(3-benzoylphenyl)propionic acid] is an active ingredient that inhibits cyclooxygenase with analgesic, antiinflammatory and antipyretic properties.

The use of ketoprofen (racemic) has been described in veterinary applications and a number of veterinary products contain ketoprofen either as tablets for oral administration for dogs or as an injectable solution for cattle, pigs and horses. Both these preparations are for strictly individual use and animals must be treated one by one both when reared individually and also when reared in herds (farms).

In modem intensive farming, non-dairy livestock, for example pigs, beef cattle, etc. are reared in large herds that can hold up to thousands of animals. In these farms, the animal population density is very high and when a focus of infection appears this rapidly spreads to the whole herd (or at least the whole stockyard), especially in the case of respiratory infections. In these cases, the whole herd should be treated simultaneously. If these are treated with the ketoprofen products currently available on the market, each individual animal must be held still and administered the appropriate dose of the medicine and the whole process repeated for several days. This is very time-consuming and requires considerable manual work, increasing greatly the cost of the treatment, which must be added to the cost of the antibiotic treatment administered simultaneously, and causing animal stress that greatly worsens the course of the illness, making this type of treatment unviable in practise.

For the regulation of the reproductive cycle of pigs, the use of a feed for livestock supplemented with a NSAID compound, such as ketoprofen is known from WO 97/45113. WO 9618298 describes a method for treating poultry which comprises the step of administering orally aspirin in the poultry feed for enhancing weight gain and preventing focal ulcerative dermatitis. In addition EP 048919 describes a method for treating leg weakness in poultry by the administration mixed with drinking water or food of a non esteroidal anti-inflamatory drug selected among Diclofenac Na, Naproxen, Ibuprofen, Nabumetone, Acetylsalicylic acid, Sulindac e Indomethacin.

### SUMMARY OF THE INVENTION

The invention addresses the problem of simultaneously treating a herd of animals for Porcine Respiratory Disease Complex (PRDC) in pigs and Bovine Respiratory Syndrome (BRC) in calves.

The solution provided by the invention is based on the inventors' observation that by adding a composition comprising ketoprofen to an animal feed in the amount required according to the weight and number of animals present, a herd of animals can be easily treated simultaneously.

A method such as that provided by this invention is advantageous because individual animals do not have to be held still to administer them the treatment, avoiding delays in administering the product and reducing manual labor costs and stress to animals.

Yet another object of the invention is the use of a composition containing ketoprofen in the production of a feed supplemented with ketoprofen for a simultaneous oral treatment of a herd of animals for Porcine Respiratory Disease Complex (PRDC) in pigs and Bovine Respiratory Syndrome (BRC) in calves.

### BRIEF DESCRIPTION OF THE FIGURES

Figure 1 is a graph showing the evolution of the rectal temperature of each group of pigs during the treatment (Example 3).
Figure 2 is a graph showing the evolution of the clinical index of each group of pigs during the treatment (Example 3).
Figure 3 is a graph showing the evolution of the clinical index of each group of calves during the treatment (Example 4).
Figure 4 is a graph showing the evolution of the rectal temperature of each group of calves during the treatment (Example 4).

### DETAILED DESCRIPTION OF THE INVENTION

The invention provides the use of a ketoprofen-based composition in the production of a feed supplemented with ketoprofen for the simultaneous oral coadjuvant treatment of Porcine Respiratory Disease Complex (PRDC) in pigs and Bovine Respiratory Syndrome (BRS) in calves.

The supplemented feed comprising ketoprofen used in the frame of this invention is a substantially solid feed that can be orally ingested by animals.

The feeds normally used for feeding animals are known products consisting of mixtures of cereals and products with a high protein content, together with additives and fillers. For the practical application of the invention any feed suitable for feeding animals can be used.

Ketoprofen is a commercially available product useful as an analgesic, anti-inflammatory and/or antipyretic agent. Alternatively, it can be obtained by a method such as that described in US Patent 3,641,127. As used in this specification, the term "ketoprofen" includes both ketoprofen as a free acid and its veterinary acceptable salts.

The supplemented feed used in the present invention contains ketoprofen in a sufficient amount to provide a therapeutically effective amount of the active ingredient to the animal ingesting the feed. As used in this specification, the term "therapeutically effective amount" refers to an amount sufficient to have a therapeutic effect on the animal, such as an amount between 1 and 5 mg of ketoprofen per kilogram live weight of the animal ingesting the feed per day (1-5 mg/kg/day).

The supplemented feed used in the frame of the invention is used for the oral treatment of PRDC in pigs and BRS in calves in a herd of animals simultaneously. As used in this specification, the term "herd of animals" refers to a group of animals, such as, for example, the group of animals present in an intensive cattle farm, and the term "simultaneously" refers to the fact that the herd of animals receives the treatment with ketoprofen at the same time, regardless of whether all the animals ingest the supplemented feed of the invention at the same time, since the ketoprofen is in the animal feed.

Example 3 shows the clinical effectiveness of a ketoprofen-based solid composition as a coadjuvant in the anti-infectious therapy of the Porcine Respiratory Disease Complex (PRDC) in groups of pigs, while Example 4 shows the clinical effectiveness of said ketoprofen-based solid composition as a complement of the anti-bacterial treatment of the Bovine Respiratory Syndrome BRS) in calves.

The supplemented feed used in the frame of the invention may be presented in different forms. In a particular embodiment, the supplemented feed is in a substantially homogeneous mixture form comprising a feed for animals and a ketoprofen-based composition comprising ketoprofen and one or more veterinary acceptable excipients. In this case, the supplemented feed can be easily prepared by mixing a feed for animals with the sufficient amount of said ketoprofen-based composition, in powder from, in order to provide the desired concentration of ketoprofen in the supplemented feed In general, when the supplemented feed in this presentation form (mixture) the necessary amount of the supplemented feed is prepared for the daily treatment of the group of animals to be treated, thereby mixing the amount of ketoprofen-based composition that provides each animal the therapeutically efficient daily amount of ketoprofen with the daily amount of feed for animals.

In another particular embodiment, the supplemented feed used in the frame of the invention is in granulated form. In this case, the supplemented feed can be obtained by mixing said ketoprofen-based composition, in powder form, as previously mentioned, with the feed for animals, subjecting the resulting mixture to a granulation process with conventional methods. When the supplemented feed is in this form (granulated), it is possible to prepare at one time the amount of supplemented feed of the invention required to carry out the complete treatment of the group of animals to be treated.

Thus, the supplemented feed used in the frame of the invention may be obtained by means of a simple method which comprises mixing a ketoprofen-based composition with feed for animals and, optionally, granulating the resulting mixture. The amount of the ketoprofen-based composition to be mixed with the animal feed may vary within a wide range, although, generally, the mixed amount of animal feed will be enough to provide the desired concentration of ketoprofen in the supplemented feed. Typically, the ketoprofen-based composition mixed with the animal feed provides between 1 and 5 mg/kg/day of ketoprofen per animal ingesting said feed.

Said ketoprofen-based composition comprises ketoprofen along with one or more veterinary acceptable excipients. In a particular embodiment, said excipients comprise one or more diluents, such as lactose, saccharose, mannitol, cellulose, xylitol, glycine, sorbitol, etc., and one or more fluidifiers, such as colloidal silicon dioxide, stearic acid, magnesium stearate, sodium stearylfumarate etc. The ketoprofen-based composition could also contain other excipients, such as flavor and/or colour additives authorized in food products.

The amount of ketoprofen in said ketoprofen-based composition may vary within a wide range, typically between 1% and 20% by weight, with respect to the total composition, preferably, between 3% and 6% by weight, with respect to the total composition. Both the lower and upper limits will be determined by the quantities of ketoprofen which are practical to prepare said ketoprofen-based composition given the intended use of the same.

By way of example, said composition presents the following formulation:

| Component | % by weight with respect to the total composition |
|---|---|
| ketoprofen (acid or salt) | 1-20% (expressed in acid) |
| Diluent | 78-98.5% |
| Fluidifier | 0.5-2% |

In a particular embodiment, said ketoprofen-based composition presents the following Formulation:

| Component | % by weight with respect to the total composition |
|---|---|
| ketoprofen | 3% |
| Lactose | 96% |
| Colloidal silicon dioxide | 1 % |

The following examples are meant for purposes of illustration of the invention.

### EXAMPLE 1

### Production of a ketoprofen-based composition

This Example was carried out with the purpose of developing a composition containing 3% ketoprofen by weight, meant for production of a feed supplemented with ketoprofen to be used in the frame of the present invention.

### 1.1 Formulation

| Component | Formulation |
|---|---|
| ketoprofen | 3.0 g |
| Colloidal silicon dioxide | 1.0 g |
| Lactose | 96.0 g |
| Total | 100.0 g |

### 1.2 Stability Test

Various batches of said Formulation were prepared and packaged in opaline/aluminium/polyethylene paper bags with the purpose of performing a stability test consisting of maintaining said bags, containing the Formulation, under normal conditions [25°C and 60% relative humidity (RH)]. The results obtained allow establishing a provisional expiration period of at least 12 months when the Formulation is kept under these conditions.

In addition, the stability of a Formulation batch after the container was opened for the first time was evaluated. The results obtained allow concluding that said Formulation may be used, at least, until 3 months after it has been opened (however, once opened, it is advisable to keep the container tightly shut).

### EXAMPLE 2

### Production of a feed supplemented with ketoprofen

This Example was carried out with the purpose of developing a supplemented feed containing 0.2% of ketoprofen by weight (60 mg ketoprofen/kg feed).

### 2.1 Supplemented feed in mixture form

400 kg of a feed supplemented with 0.2% ketoprofen were prepared mixing 800 g of the Formulation from Example 1 with 399.2 kg of "pig diet" feed or with 399.2 kg of "calf diet" feed, the composition of which is shown in Table 1, shaking until a substantially full homogenisation.

**Table 1**

| **Feed Characteristics** | | |
|---|---|---|
| **Component** | **Pig diet (%)** | **Calf diet (%)** |
| Wheat | 10.08 | - |
| Com | 7.10 | 30.30 |
| Barley | 48.02 | 30.00 |
| Lard | 3.50 | 3.00 |
| Soy 44 | 28.96 | 3.17 |
| DL-Methionin | 0.06 | - |
| Calcium carbonate | 0.95 | 1.59 |
| bicalcium phosphate | 0.56 | 1.11 |
| Salt | 0.33 | 0.30 |
| Alfalfa | - | 14.00 |
| Sunflower | - | 16.13 |
| Mineral vitamin corrector* | 0.40 | 0.40 |

| Estimated nutrient. composition | Pig diet (%) | Calf diet (%) |
|---|---|---|
| EMA (Kcal) | 3,100 | 2,709 |
| Protein | 21.1 | 15.2 |
| Fibre | 4.5 | 9.3 |
| Fat | 5.4 | 5.4 |
| Ashes | 4.8 | 7.1 |

| | | |
|---|---|---|
| * Mineral vitamin corrector: Pig diet: Per kg of feed: Vitamin A: 5000 UI, Vitamin D3: 1000 UI, Vitamin E: 15 mg, Vitamin B1: 1.3 mg, Vitamin B2: 3.5 mg, Vitamin B12: 0.025 mg, Vitamin B6: 1.5 mg, Calcium panthotenate: 10 mg, Nicotinic acid: 15 mg, Biotin: 0.1 mg, Folic acid: 0.6 mg, Vitamin K3: 2 mg, Fe: 80 mg, Cu: 6 mg, Co: 0.75 mg, Zn: 60 mg, Mn: 30 mg, I: 0.75 mg, Se: 0.10 mg and Ethoxyquin: 0.15 mg. Calf diet: Per kg of feed: Vitamin A: 10000 Ul, Vitamin D3: 2000 Ul, Vitamin E: 10 mg, Cu: 2 mg, Co: 1 mg, Zn: 80 mg, Mn: 50 mg, Mg: 180 mg, I: 1 mg, Se: 0.25 mg, anhydrous sodium sulphate: 250 mg and Ethoxyquin: 125 mg. | | |

Once the feed supplemented with 0.2% ketoprofen in mixture form was obtained, various samples were taken to carry out the corresponding tests.

### 2.1.1 General Characteristics

The supplemented feed, as previously obtained, was analyzed to evaluate its appearance and water content and also to determine the presence and content of ketoprofen.

### Appearance:

Pig diet: Brown colour heterogeneous mixture in all cases
Calf diet: Dark brown colour heterogeneous mixture in all cases

### Water content by Karl-Fischer (%) (water from the humidity in the feed):

| **Feed** | **Sample 1** | **Sample 2** | **Sample 3** | **Average** |
|---|---|---|---|---|
| Pig diet | 7.3 | 7.6 | 7.8 | 7.6 |
| Calf diet | 7.5 | 7.5 | 7.9 | 7.6 |

Presence of ketoprofen by HPLC: Positive in all cases

### Content of ketoprofen by HPLC (%):

| **Feed** | **Sample 1** | **Sample 2** | **Sample 3** | **Average** |
|---|---|---|---|---|
| Pig diet | 98.1 | 93.1 | 93.8 | 95.0 |
| Calf diet | 91.0 | 84.8 | 77.8 | 84.5 |

The results obtained allow concluding that the mixing process leads to obtaining a reasonably homogeneous mixture.

### 2.1.2 Stability test

Various batches of previously obtained feed supplemented with ketoprofen (mixture) were prepared and were packaged in polyethylene jars with the purpose of carrying out a stability test consistent in maintaining said jars in normal conditions [25°C and 60% RH]. The results obtained allow the establishment of a provisional expiration period of, at least, 2 months when said supplemented feed are kept under the established conditions.

### 2.1.3 Transportation test

The effect of transporting a 75 kg sack of each of the supplemented feed during approximately 350 km on the possible disaggregation of the mixture was studied. For this purpose, samples were analyzed executing this transportation in order to verify its influence. The results obtained (by % ketoprofen) are shown below:

| **Feed** | **Sample 1** | **Sample 2** | **Sample 3** | **Average** |
|---|---|---|---|---|
| Pig diet | 83.6 | 86.1 | 92.6 | 87.4 |
| Calf diet | 95.7 | 96.5 | 94.5 | 95.6 |

The results obtained show that no disaggregation was observed.

### 2.2 Supplemented feed in granulated form

300 kg of each of the mixtures obtained in Example 2.1 were subjected to a granulation process in a Mabrik PV-30 granulation press, by vapour action at a final temperature of 65°C for approximately 20 minutes, with subsequent cooling at room temperature, proceeding then to the sampling for each granulation to determine their general characteristics and stability.

### 2.2.1 General Characteristics

The results relative to appearance, water content, and also to presence and content of ketoprofen are shown below.

### Appearance:

Pig diet: Brown colour cylindrical granules in all cases
Calf diet: Dark brown colour cylindrical granules in all cases

### Water content by Karl-Fischer (%):

| **Feed** | **Sample 1** | **Sample 2** | **Sample 3** | **Average** |
|---|---|---|---|---|
| Pig diet | 7.9 | 7.9 | 8.8 | 8.2 |
| Calf diet | 7.5 | 7.9 | 8.0 | 7.8 |

Presence of ketoprofen by HPLC: Positive in all cases

### Content of ketoprofen by HPLC (%):

| **Feed** | **Sample 1** | **Sample 2** | **Sample 3** | **Average** |
|---|---|---|---|---|
| Pig diet | 85.0 | 83.2 | 82.4 | 83.5 |
| Calf diet | 84.0 | 81.8 | 81.2 | 82.3 |

The results obtained allow stating that the granulation process leads to the production of a reasonably homogeneous granulation.

### 2.2.2 Stability Study

Various batches of the previously obtained feed supplemented with ketoprofen (granulated) were prepared and packaged in polyethylene jars with the purpose of conducting a stability test consisting of maintaining said jars under normal conditions [25°C and 60% RH]. The results obtained allow establishing a provisional expiration period of, at least, 2 months when said supplemented feed are kept under the established conditions.

### EXAMPLE 3

### Study of the clinical effectiveness of a ketoprofen-based composition, orally administered by means of a feed (feed supplemented with ketoprofen), as a complement of the antibiotic treatment of the Porcine Respiratory Disease Complex

A controlled, randomized and blind clinical trial was conducted out in accordance with the principles of the Good Clinical Practice, in order to evaluate the clinical effectiveness of the 3% ketoprofen-based composition (Example 1), from now on EV-4556, following its oral administration by means of a feed (supplemented feed) as a coadjuvant treatment to the antibacterial treatment of the Porcine Respiratory Disease Complex (PRDC) in groups of pigs.

The study took place with a total of 77 pigs of mixed race and different sex belonging to two farms where an outbreak of PRDC had been diagnosed. At the beginning of the experimental phase they were distributed in two treatment groups in a random fashion with similar size (Groups A and B).

Next, the animals of both groups received an intramuscular antibacterial treatment (enrofloxacin) for 3 consecutive days. During this same period, the animals in one of the two groups received, in addition, the EV-4556 composition mixed in the feed in a ratio of 1g/10kg live weight/day (equivalent to 3 mg/kg/day of ketoprofen) and the animals in the other group received 1g/10kg live weight/day of a placebo (the excipient for the EV-4556 composition).

The experimental period of the study lasted 5 days (days D0, D1, D2, D3 and D4) during which, in addition to selecting the animals and administering the treatments (days D0 to D3), a clinical monitoring of the animals by periodic controls (days D1, D2, D3 and D4) took place. In the course of these controls the rectal temperature of each of the animals was measured and various clinical parameters were given a score, based on which a clinical index showing the level of disease was calculated.

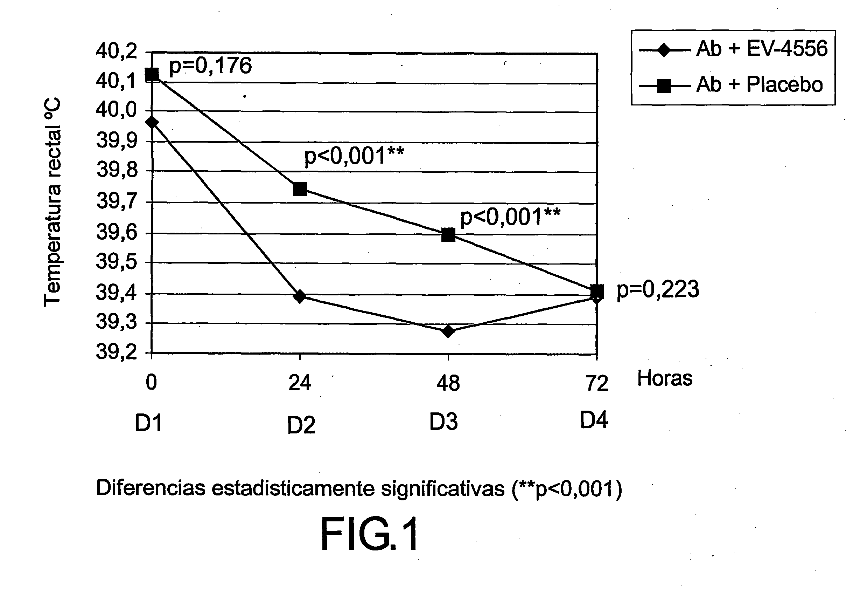

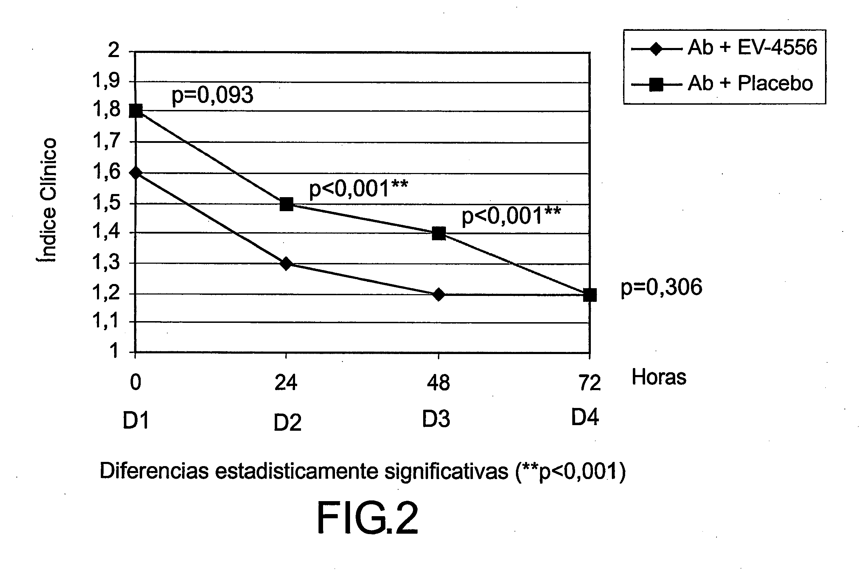

The results on the evolution of the rectal temperature are shown in Table 2 and Figure 1, while the results obtained on the evolution of the clinical index are shown in Table 3 and in Figure 2.

**Table 2**

| **Evolution of rectal temperature** | | | |
|---|---|---|---|
| | **GROUP A (Ab + EV-4556) n=39** | **GROUP B (Ab + placebo) n=38** | **Statistical comparison** |
| **D1 (0h)** | 40.0 ± 0.4 °C | 40.1 ± 0.5 °C | p = 0.176 |
| **D2 (24h)** | 39.4 ± 0.4 °C | 39.7 ± 0.4 °C | p < 0.001** |
| **D3 (48h)** | 39.3 ± 0.4°C | 39.6 ± 0.3°C | P <0.001** |
| **D4 (72h)** | 39.4 ± 0.5°C | 39.4 ± 0.3 °C | p= 0.223 |

The average values ± Standard deviation of the rectal temperature of each group during the course of the treatment are shown.
Statistically significant differences (**p < 0.001)

**Table 3**

| **Evolution of the Clinical Index** | | | |
|---|---|---|---|
| | **GROUP A (Ab + EV-4556) n=39** | **GROUP B (Ab + placebo) n=38** | **Statistical comparison** |
| **D0** | 1.6 ± 0.4 | 1.8 ± 0.5 | p = 0.093 |
| **D2** | 1.3 ± 0.2 | 1.5 ±0.4 | P <0.001** |
| **D3** | 1.2 ± 0.2 | 1.4 ± 0.3 | P <0.001** |
| **D4** | 1.2 ± 0.3 | 1.2 ± 0.3 | p = 0.306 |

The average values ± standard deviation of the Clinical Index for each group during the course of the treatment are shown.
Statistically significant differences (**p<0.001)

Throughout the treatment, both the clinical index and the rectal temperature of the animals decreased much more significantly in Group A (treated with the antibiotic (Ab) along with EV-4556) than in Group B (treated with antibiotic and placebo), the differences observed between both groups being, in addition, statistically significant both in the control that took place 24 hours into the treatment (control day D2) and in the control that took place 48 hours into the treatment (control day D3).

The results of the study show that the EV-4556 composition, administered mixed in the feed at a dosage of 1g/10kg/day (equivalent to 3 mg/kg/day of ketoprofen) during 3 consecutive days, is efficient as a coadjuvant treatment in the antiinfectious therapy of the Porcine Respiratory Disease Complex, contributing to the acceleration of the remission of the symptoms and, thus, to the clinical recovery of the animal.

### EXAMPLE 4

### Study of the clinical effectiveness of a ketoprofen-based composition, orally administered by means of a feed (feed supplemented with ketoprofen), as a complement of the antibacterial treatment of the Bovine Respiratory Syndrome (BRS) in calves

A multicentric, controlled, randomized and blind clinical study was conducted, in compliance with Good Clinical Practice principles, with the purpose of evaluating the clinical effectiveness of the 3% ketoprofen composition (Example 1), from now on, EV-4556, following its oral administration by means of a supplemented feed, as a coadjuvant treatment in the antibacterial treatment of the Bovine Respiratory Syndrome (BRS) in groups of calves.

The study was performed with a total of 338 nursing calves of different race and sex, with symptoms compatible with BRS. At the beginning of the experimental phase they were distributed into two treatment groups in a random fashion and in similar size.

All animals received a treatment with an antibacterial drug (two administrations of 20 mg of Florfenicol/kg in an interval of 48 hours). Additionally, the animals of one of the two groups received a dosage of 3 mg/kg/day of ketoprofen during 3 consecutive days by administration of the EV-4556 composition. The animals of the other group received equivalent quantities of a placebo, consisting of the excipient of the EV-4556 composition, also during 3 consecutive days. Both products (EV-4556 and placebo) were administered orally, mixed in the feed.

The experimental period of the study lasted 10 days, during which the treatment administration (days 1 to 3), a clinical monitoring of the animals (days 1 to 4) and a monitoring of the possible appearance of recidivations (days 4 to 10) all took place. The daily clinical monitoring of the animals consisted on evaluating the level of disease by calculating the clinical index, as well as by registering the rectal temperature of the animals.

The results of the study showed that the success percentage of the treatment based on antibiotic + EV-4556 composition was significantly greater than the treatment based on antibiotic + placebo. Specifically, the success percentage in the group that was treated with antibiotic + EV-4556 once completed the treatment was 93.5%, while in the group treated with antibiotic + placebo it was only 72.2%, these differences being statistically significant. Furthermore, from the second day of treatment the average value of the clinical index was significantly lower in the group that was treated with the antibiotic + EV-4556 composition that in the group treated with antibiotic + placebo (days 2, 3 and 4; p<0.001), showing that the decrease in symptoms and, thus, the cure of the illness had taken place in a significantly quicker manner when the EV-4556 composition was administered. On the other hand, the average value of the rectal temperature of the animals throughout the treatment was also significantly lower in the first group than in the second (p<0.001).

In short, the results of the study led to the conclusion that the EV-4556 composition has a very high clinical effectiveness as a coadjuvant treatment in the antibacterial therapy of Bovine Respiratory Syndrome in calves.

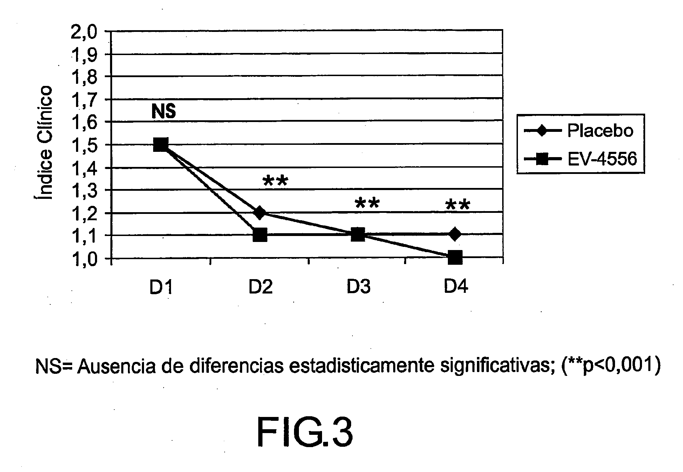

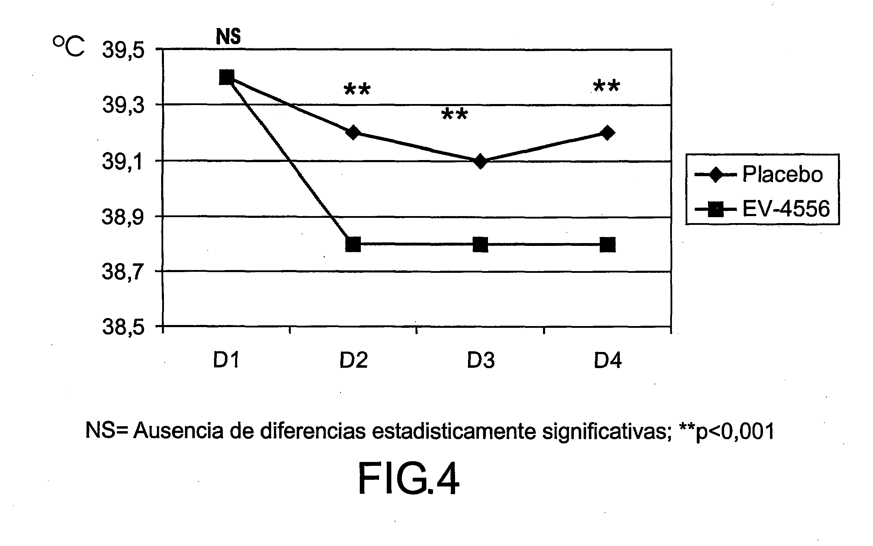

The results of the evolution of the clinical index are shown in Table 4 and Figure 3, while those of the percentage of animals in which the treatment was evaluated as a success or failure in each of the groups tested are shown in Table 6 and the results of the evolution of rectal temperature are shown in Table 6 and in Figure 4.

**Table 4**

| **Evolution of Clinical Index** | | | |
|---|---|---|---|
| | **GROUP A (placebo)** | **GROUP B (EV-4556)** | **Statistical comparison** |
| **D1** | 1.5 ± 0.3 | 1.5 ± 0.3 | NS |
| **D2** | 1.2 ± 0.2 | 1.1 ± 0.1 | p<0.001 |
| **D3** | 1.1 ± 0.2 | 1.1 ± 0.1 | p<0.001 |
| **D4** | 1.1 ± 0.2 | 1.0 ± 0.1 | p<0.001 |

The average values ± standard deviation of the Clinical Index of each group during the course of the treatment are shown.
NS = No statistically significant differences

**Table 5**

| **Percentage of animals in which the treatment was evaluated as a success or a failure in each of the groups** | | | |
|---|---|---|---|
| | **GROUP A (placebo)** | **GROUP B (EV-4556)** | **Statistical comparison** |
| **SUCCESS Clinical index on day D4** ≤ **1.1** | 122 (72.2%) | 158 (93.5%) | p<0.001^{#} |
| **FAILURE Clinical index on day D4 > 1.1** | 47(27.8%) | 11(6.5%) | |

| | | | |
|---|---|---|---|
| ^{#}Chi-squared Test = 26.973 with 1 degree of freedom. Statistical power=100% | | | |

**Table 6**

| **Evolution of rectal temperature** | | | |
|---|---|---|---|
| | **GROUP A (placebo)** | **GROUP B (EV-4556)** | **Statistical comparison** |
| **D1** | 39.4 ± 0.6 °C | 39.4 ± 0.6 °C | NS |
| **D2** | 39.2 ± 0.4 °C | 38.8 ± 0.3 °C | p<0.001 |
| **D3** | 39.1 ± 0.4°C | 38.8 ± 0.4 °C | p<0.001 |
| **D4** | 39.2 ± 0.4 °C | 38.8 ± 0.4 °C | p<0.001 |

## Claims

1. Use of a ketoprofen-based composition in the production of a feed supplemented with ketoprofen, for the simultaneous oral coadjuvant treatment to antibacterial treatment of Porcine Respiratory Disease Complex (PRDC) in pigs and Bovine Respiratory Syndrome (BRS) in calves.

2. Use according to claim 1, wherein said ketoprofen-based composition comprises ketoprofen together with one or more veterinary acceptable excipients selected among diluents and fluidifiers.

3. Use according to any of the previous claims, wherein said ketoprofen-based composition comprises one or more diluents selected among lactose, saccharose, mannitol, cellulose, xylitol, glycine, sorbitol, and one or more fluidifiers selected among colloidal silicon dioxide, stearic acid, magnesium stearate and sodium stearylfumarate.

4. Use according to any of the previous, claims, wherein said ketoprofen-based composition presents the following composition:
| Component | % by weight with respect to the total composition |
|---|---|
| ketoprofen (acid or salt) | 1-20% (expressed in acid) |
| Diluent | 78-98.5% |
| Fluidifier | 0-5-2% |

5. Use according to claim 4, wherein said ketoprofen-based composition presents the following composition:
| Component | % by weight with respect to the total composition |
|---|---|
| ketoprofen (acid or salt) | 3% (expressed in acid) |
| Lactose | 96% |
| Colloidal silicon dioxide | 1 % |

## Patentansprüche

1. Verwendung einer auf Ketoprofen basierenden Zusammensetzung bei der Herstellung von Futter, das mit Ketoprofen ergänzt ist, für die simultane, orale, adjuvante Behandlung zur antibakteriellen Behandlung des porcinen respiratorischen Krankheitskomplexes (PRDC, englisch: Porcine Respiratory Disease Complex) bei Schweinen und des bovinen respiratorischen Syndroms (BRS) bei Kälbern.

2. Verwendung gemäß Anspruch 1, wobei die besagte auf Ketoprofen basierende Zusammensetzung Ketoprofen zusammen mit einem oder mehreren veterinärmedizinisch annehmbaren Vehikeln, ausgewählt unter Verdünnungsmitteln und Substanzen zum Fließfähigmachen (englisch: fluidifiers), umfasst.

3. Verwendung gemäß einem der vorhergehenden Ansprüche, wobei die besagte auf Ketoprofen basierende Zusammensetzung ein oder mehrere Verdünnungsmittel, ausgewählt unter Lactose, Saccharose, Mannit, Cellulose, Xylit, Glycin, Sorbit, und eine oder mehrere Substanzen zum Fließfähigmachen, ausgewählt unter kolloidalem Siliciumdioxid, Stearinsäure, Magnesiumstearat und Natriumstearylfumarat, umfasst.

4. Verwendung gemäß einem der vorhergehenden Ansprüche, wobei die besagte auf Ketoprofen basierende Zusammensetzung die folgende Zusammensetzung aufweist:
| Bestandteil | Gewichtsprozent in Bezug auf die Gesamtzusammensetzung |
|---|---|
| Ketoprofen (Säure oder Salz) | 1-20 % (in Säure ausgedrückt) |
| Verdünnungsmittel | 78-98,5 % |
| Substanz zum Fließfähigmachen | 0,5-2 % |

5. Verwendung gemäß Anspruch 4, wobei die besagte auf Ketoprofen basierende Zusammensetzung die folgende Zusammensetzung aufweist:
| Bestandteil | Gewichtsprozent in Bezug auf die Gesamtzusammensetzung |
|---|---|
| Ketoprofen (Säure oder Salz) | 3 % (in Säure ausgedrückt) |
| Lactose | 96 % |
| Kolloidales Siliciumdioxid | 1 % |

## Revendications

1. Utilisation d'une composition à base de kétoprofène pour la production d'un aliment enrichi en kétoprofène, simultanément en tant que traitement d'appoint oral pour un traitement antibactérien du complexe de la maladie respiratoire porcine (PDRC) des porcs et du syndrome respiratoire bovin (BRS) des veaux.

2. Utilisation selon la revendication 1, dans laquelle ladite composition à base de kétoprofène comprend du kétoprofène en même temps que un ou plusieurs excipients acceptables d'un point de vue vétérinaire, choisis parmi les diluants et fluidifiants.

3. Utilisation selon l'une quelconque des revendications précédentes, dans laquelle ladite composition à base de kétoprofène comprend un ou plusieurs diluants choisis dans le groupe comprenant lactose, saccharose, mannitol, cellulose, xylitol, glycine, sorbitol et un ou plusieurs fluidifiants choisis dans le groupe comprenant dioxyde de silicium colloïdal, acide stéarique, stéarate de magnésium et stéaryl-fumarate de sodium.

4. Utilisation selon l'une quelconque des revendications précédentes, dans laquelle ladite composition à base de kétoprofène présente la composition suivante:
| Composant | % en poids par rapport à la composition totale |
|---|---|
| Kétoprofène (acide ou sel) | 1 à 20% (exprimé en acide) |
| Diluant | 78 à 98,5% |
| Fluidifiant | 0,5 à 2% |

5. Utilisation selon la revendication 4, dans laquelle ladite composition à base de kétoprofène présente la composition suivante:
| Composant | % en poids par rapport à la composition totale |
|---|---|
| Kétoprofène (acide ou sel) | 3 % (exprimé en acide) |
| Lactose | 96 % |
| Dioxyde de silicium colloïdal | 1 % |
